(19) **Europäisches Patentamt**
**European Patent Office**
**Office européen des brevets**

(11) **EP 4 725 498 A1**

(12) **EUROPEAN PATENT APPLICATION**

(43) Date of publication:
**15.04.2026 Bulletin 2026/16**

(21) Application number: **24206224.8**

(22) Date of filing: **11.10.2024**

(51) International Patent Classification (IPC):
**A61K 39/00** (2006.01)   **C07K 14/435** (2006.01)
**A61P 33/14** (2006.01)   **A61P 33/00** (2006.01)

(52) Cooperative Patent Classification (CPC):
**C07K 14/43527; A61K 39/0003; A61P 33/14;**
A61K 2039/552; A61K 2039/55566; A61K 2039/575;
A61K 2039/70; C07K 2319/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **La Buena Estrella SA**
**14000 Canelones (UY)**

(72) Inventors:
• **SANGUINETTI ACOSTA, Carlos Julio**
**11100 Montevideo (UY)**
• **RODRIGUEZ AUGE, Sebastian Marcelo**
**11100 Montevideo (UY)**
• **BETANCOR DUTRENIT, Lorena**
**11100 Montevideo (UY)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **VACCINE COMPOSITION COMPRISING A CHIMERIC PROTEIN AGAINST RHIPICEPHALUS MICROPLUS**

(57)    The present invention refers to a vaccine composition comprising one or more tick's proteins or antigens, preferably selected from the list consisting of the Bm86 protein, Subolesin protein, and/or P0 protein. The present invention also refers to uses of said vaccine compositions to generate an immune response against ticks, and kits comprising the same.

## Description

### TECHNICAL FIELD

The present invention relates to the field of vaccinology. Particularly, the present invention relates to the field of vaccines against ticks.

### BACKGROUND ART

Ticks, mites of the suborder Ixodida, are ectoparasites that cause the most losses in the agricultural sector worldwide, affecting 80% of the global livestock population.

The economic impact is directly related to the epidemiology of infestation and can result in direct or indirect losses. Their direct effects on production include damage to the skin from bites, blood loss associated with high parasite burdens, severe immunological reactions due to toxin injection, permanent stress affecting animal behavior and well-being, and energy loss associated with constant movement in response to infestation [1,2].

Indirect losses are related to the effects of hemoparasites and other diseases transmitted by ticks [2]. Examples of these include the cost of treating clinical cases, expenses for parasite control, lost profits, the use of genetically tick-resistant but less productive breeds, loss of meat or milk due to acaricide residues, and restrictions on animal trade between areas or countries.

Losses caused by *Rhipicephalus microplus* infestation and its control have been estimated by some authors to be between USD $13.9-18.7 billion annually globally.

In Uruguay, the species *Rhipicephalus microplus* has the greatest economic impact, resulting in annual losses of 32 to 45 million dollars due to expenses on acaricides, weight loss, treatment costs, deaths from tick-borne diseases, and campaign costs. Additionally, there may be market losses due to the presence of insecticide residues in meat and milk.

Over time, the control of this parasite has been carried out using acaricides and herd management. However, these methods have not achieved the desired success, mainly due to the high proliferation of the parasite and the development of resistance to different acaricides. Acaricides are expensive, can leave residues in meat or dairy products, can cause environmental contamination, and often lead to resistance. As a result, alternative methods for tick control are currently being sought.

One potentially more effective and sustainable alternative to the use of chemicals is the biotechnological approach through vaccination. Since the 1990s, there have been two commercially available recombinant vaccines worldwide (Gavac® from Heber Biotec, Havana, Cuba, and Tick Gard® from Hoechst Animal Health, Australia) that use a protein called Bm86 as an immunogen. This protein is located on the luminal surface of the tick's midgut, and when it is blocked by antibodies generated through vaccination in cattle, it causes the lysis of the tick's gut wall, interfering with blood digestion and egg production [2].

However, the efficacy of these vaccines has been modest, and even poor when applied in countries where they have not been developed, leading to low levels of vaccine acceptance in the market. Tick Gard® is no longer commercially available, and Gavac® [2,4] has limited availability. In recent years, research efforts have focused on identifying and developing new antigens that provide more effective protection.

The failure of these vaccines' efficacy, both in Uruguay and in other countries in the region, varies between 51% and 91%, and these results have been associated with amino acid divergences in the Bm86 protein [4].

These results have led to the production of region-specific recombinant antigen Bm86 and the search for new antigenic combinations to develop effective immunity.

Bm86 is not the only described antigen that functions in tick infestation protection. The exposed antigen Subolesin is located in the salivary glands and is associated with functions such as transcription, immunogenicity, gene expression, development, and physiology [3,5]. On the other hand, the antigen P0 is a protein located in the cellular cytoplasm and is essential for the assembly of the 60s ribosomal subunit. Blocking this protein is associated with the inhibition of protein synthesis and subsequent cell death. The poor performance of current vaccines has prompted researchers and technologists to seek alternatives for the development of new vaccines. A more recent approach to improve protection in vaccines in general, including tick vaccines, is the use of chimeric proteins, which are non-natural proteins formed by sections of other proteins that fuse different epitopes [4]. The antigenic nucleotide sequences are fused using a linker sequence and inserted into an expression plasmid. Although this technology shows promise, reports in the scientific literature on the use of chimeric vaccines are scarce, and there are no approaches that can lead to commercialization [6].

The use of combinations of target antigens to combat tick infestation has been reported in academic literature as a strategy to enhance the immune response [4,2]. Compared to other vectors and pathogens, ticks have very large genomes ranging from 2.1 to 7.1 Gb. Additionally, they are highly repetitive, which may be crucial for tick survival. Recent literature reviews indicate that the use of antigenic cocktails has shown improved efficacy [3,4]. However, generating vaccines comprising antigenic cocktails comes with several drawbacks, such as 1) the different antigens will compete for B and T cell stimulation, with some antigens being more immunogenic or immunodominant than others, 2) higher costs of production 3) need for stronger adjuvants, 4) cross-reactivity between antigens, 5) pathogen evolution, 6) increased time of manufacture, 7) storage and stability impacts.

In the current invention, a multivalent recombinant vaccine has been generated, composed of several antigens: BM86s, Subolesin, P0, and a *de novo* synthesized chimeric protein based on other proteins with potential antigenicity (Subolesin-P0, SPOS), with amino acid sequences that better match the Uruguayan population.

## BRIEF DESCRIPTION OF DRAWINGS

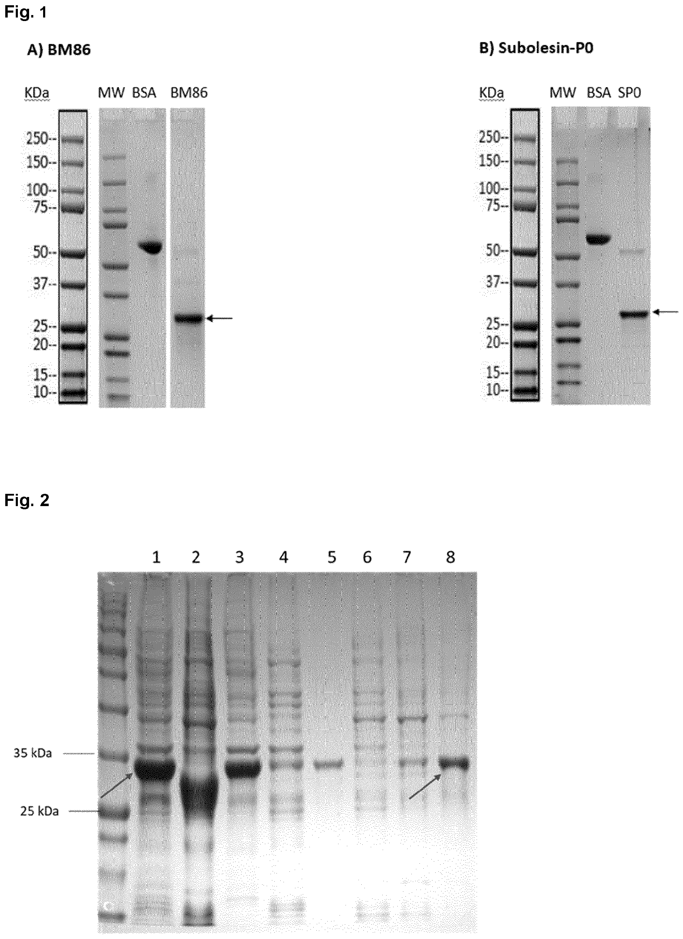

**Fig. 1** Purified recombinant BM86s and SPOS produced in *E.coli* separated in 12% polyacrylamide gels. A) BM86s; B) SPOS. Our target antigens are indicated with black arrows. Abbreviation: MW, molecular weight marker (Bio-rad, Cat. No. 1610374S, refer to annotated key on the left for size in KDa), BSA, Bovine Serum Albumin protein control (66.5 kDa).

**Fig. 2** Protein visualization with Coomassie staining. SDS-PAGE analysis (12%) of the recombinant expression and partial purification of BM86s antigen. Molecular weight marker (AccuRuler RGB PLUS Prestained Protein Ladder 02102-250), 1- inclusion bodies BM86s 1/10, 2- Inclusion bodies applied to IDA-Ni column, 3- Pellet post lysis buffer solubilization (1/10), 4- flowthrough from IDA-Ni, 5- IDA-Ni pre-elution (1/10), 6-Wash, 7- Elution with 250 mM imidazol, 8- Elution with 500 mM imidazol. The target antigen is highlighted with an arrow.

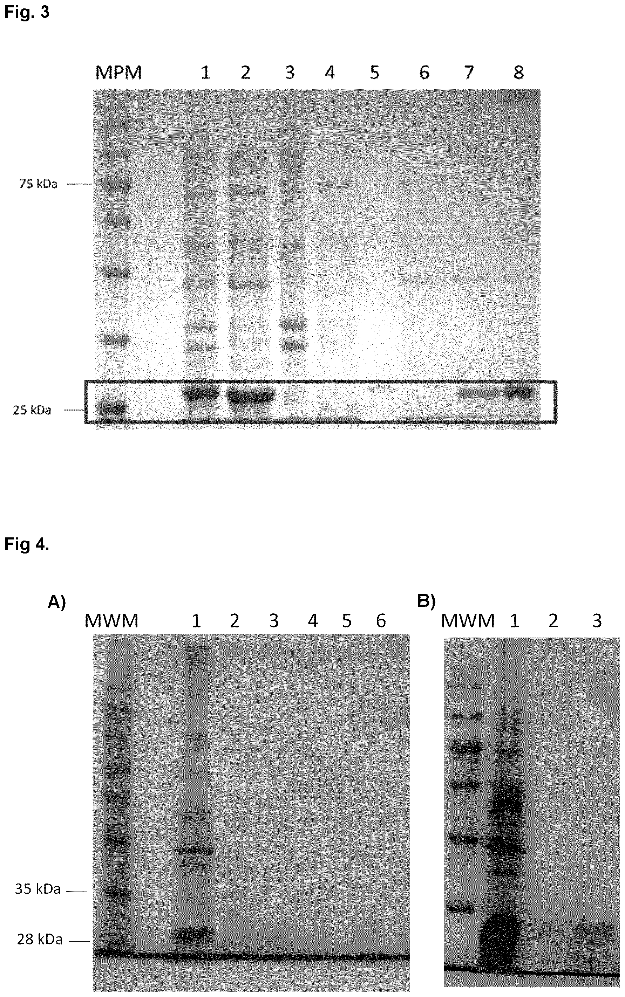

**Fig. 3** Protein visualization was achieved using Coomassie staining. SDS-PAGE analysis (12%) of the recombinant expression and partial purification of recombinant SPOS. Molecular weight marker (AccuRuler RGB PLUS Prestained Protein Ladder 02102-250), 1- inclusion bodies BM86s 1/10, 2- Inclusion bodies applied to IDA-Ni column, 3-Pellet post lysis buffer solubilization (1/10), 4- flowthrough from IDA-Ni, 5-IDA-Ni pre elution (1/10), 6- Wash, 7- Elution with 250 mM imidazol, 8- Elution with 500 mM imidazol. The target antigen is highlighted with a box.

**Fig. 4** Protein visualization was achieved using Silver staining. SDS-PAGE analysis (12%) of the final purified recombinant A) SPOS and B) BM86s. 1) MWM- AccuRuler RGB PLUS Prestained Protein Ladder 02102-250, 2- Eluted pool from IDA-Ni column using 250mM and 500 mM imidazole. The following lanes correspond to gel filtrated purified samples of the antigens.

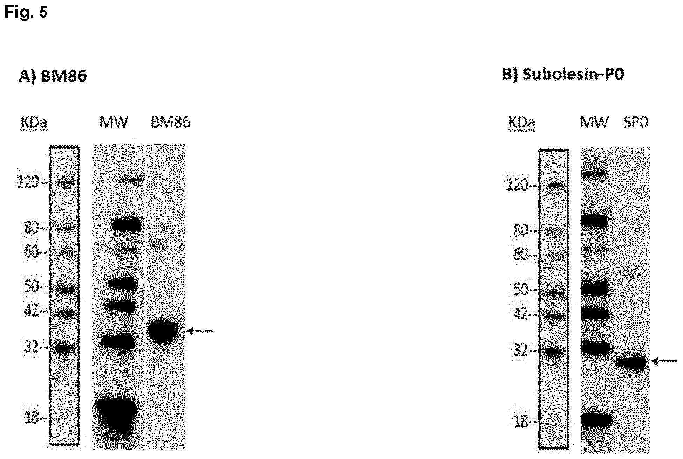

**Fig. 5:** Detection of recombinant A) BM86s and B) SPOS by Western blot analysis, using mouse-anti-His mAb (GenScript). BM86s and SPOS antigens are indicated with black arrows. Abbreviation: MW, molecular weight marker (GenScript, Cat. No. M00673, refer to annotated key on the left for size in KDa).

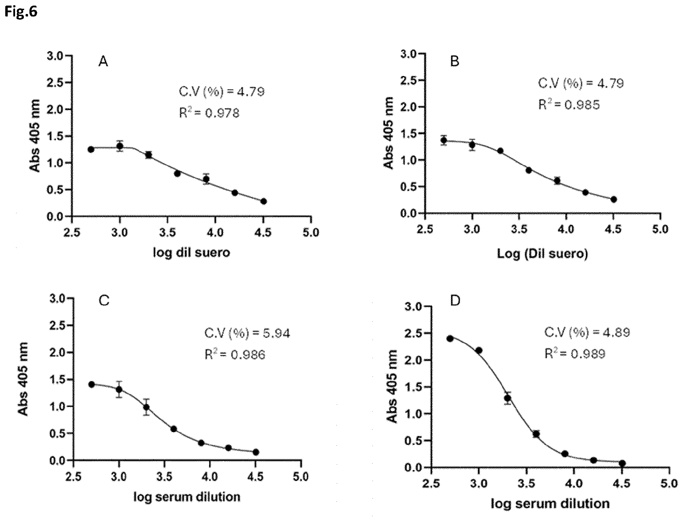

**Fig. 6:** Evaluation of the immune response in serum of vaccinated cattle after 1 dose + 2 boosters using the indirect ELISA assay. Antibody titers in immunized cattle were expressed as the $OD_{405nm}$ value as a function of serial dilutions of serum samples (10 logarithm of the dilution factor of the serum. Values were plotted with an asymmetric sigmoidal fit. A) Immune response corresponding to the subolesin antigen, B) Immune response corresponding to the BM86s antigen, C) Immune response corresponding to the SPOS antigen and D) Immune response corresponding to the BM86s - SPOS antigen.

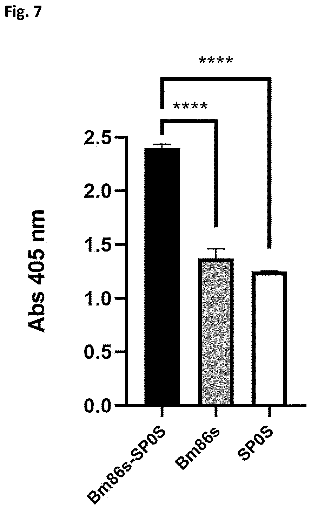

**Fig. 7:** Statistical analysis of the response obtained from the pool of sera in the ELISA test for the antigen cocktail (BM86s and SPOS) in black, BM86s in grey and SPOS in white at 1/500 serum dilution. The bar graph represents mean $\pm$ sd, n=2. Unpaired T-test (**$p < 0.01$). Two-way ANOVA, followed by Tukey's multiple comparisons test ($p < 0.05$, **$p < 0.01$, ***$p < 0.0001$).

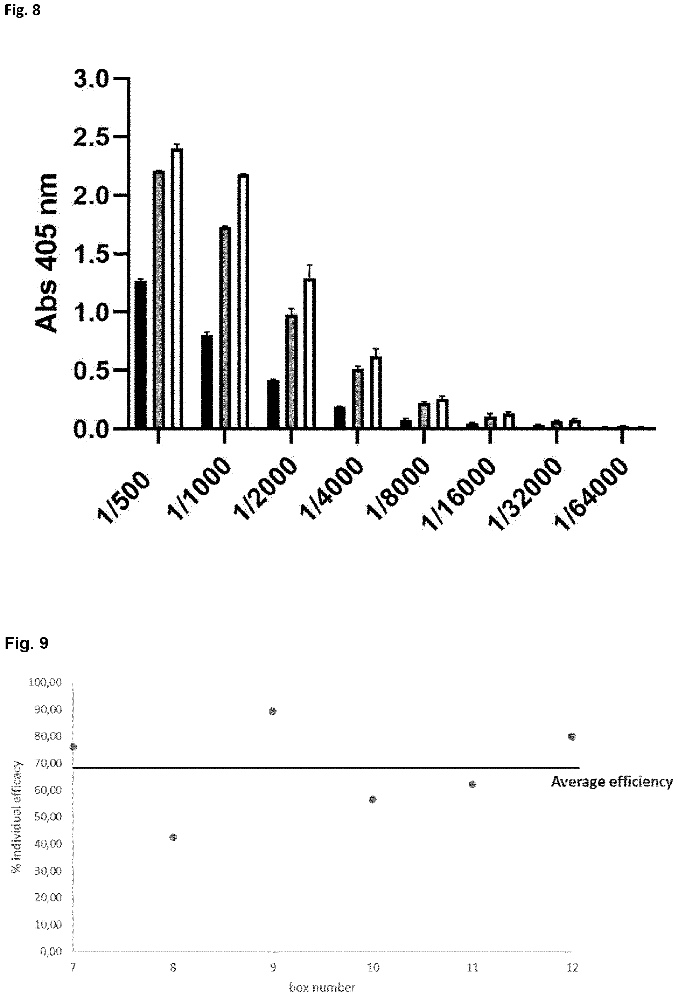

**Fig. 8:** Longitudinal ELISA response obtained for immunized cattle with antigenic cocktail over time. The X axis corresponds to sera dilutions. Columns in black correspond to the first dose; columns in grey correspond to the first dose and one booster; Columns in white corresponds to the first dose and two boosters.

**Fig. 9:** Individual efficacy of the cocktail vaccine in cattle (n=6, housed in boxes 7-12) after one dose and two boosters of BM86s and SPOS. The correlation with the serum response was evaluated by indirect ELISA.

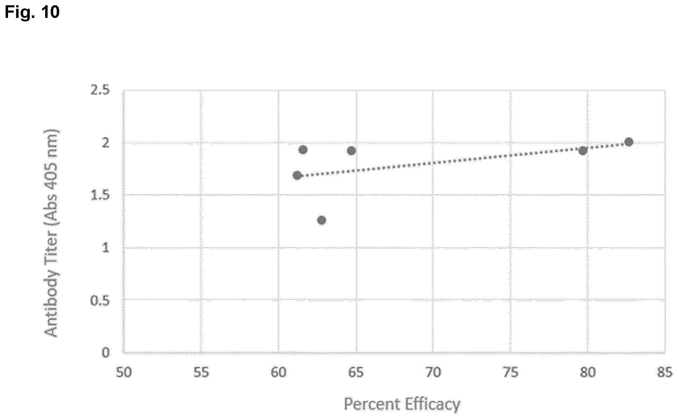

**Fig 10:** Correlation of potency (percent efficacy) and antibody titers as measured by ELISA (Abs 405nm) for individual cows (n=6).

**GENERAL DEFINITIONS**

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity indicates that a number can vary between ± 20 % around its indicated value. Preferably "about" means ± 15 % around its value, more preferably "about" means ± 10, 8, 6, 5, 4, 3, 2 % around its value, or even "about" means ± 1 % around its value, in that order of preference.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of', though less preferred.

When used herein "consisting of' excludes any element, step, or ingredient not specified in the claim element. When used herein, "consisting essentially of" does not exclude materials or steps that do not materially affect the basic and novel characteristics of the claim.

The terms "sequence identity" or "percent identity" in the context of two or more nucleotide sequences, polypeptide sequences or proteins sequences refers to two or more sequences or subsequences that are the same ("identical") or have a specified percentage of nucleotide or amino acid residues that are identical ("percent identity") when compared and aligned for maximum correspondence with a second molecule, as measured using a sequence comparison algorithm (e.g., by a BLAST alignment, or any other algorithm known to persons of skill), or alternatively, by visual inspection. The "sequence identity" or "percent identity" can be determined by calculating the number of identical nucleotides or amino acids at the same positions in a nucleic acid, polypeptide or protein. Calculation of percent identity includes determination of the optimal alignment between two or more sequences. Alignment for purposes of determining percent amino acid sequence identity can be achieved in various ways that are within the skill in the art, for example, using publicly available computer software such as BLAST, ALIGN, or Megalign (DNASTAR) software. Those skilled in the art can determine appropriate parameters for measuring alignment, including any algorithms needed to achieve maximum alignment over the full-length of the sequences being compared. Preferably, the algorithm used to calculate the percentage of identity of two or more sequences is a local alignment algorithm, such as the one used by BLAST.

By "antigen" or "immunogen" is meant a substance that induces a specific immune response in a host animal. The presence of antigens in the body normally triggers an immune response. Thus, antigens are "targeted" by antibodies. "Epitope" refers to the specific antigenic determinant of an antigen. An epitope could comprise three amino acids in a spatial conformation which is unique to the epitope. Generally, an epitope consists of at least five such amino acids, and more usually consists of at least 8-10 such amino acids. Methods of determining the spatial conformation of such amino acids are known in the art.

A subunit vaccine is a vaccine that presents one or more antigens to the immune system without introducing pathogen particles, whole or otherwise. By "protein subunit vaccine" is referred herein as to specific isolated antigens from a pathogen, preferably a tick. A "protein subunit vaccine" is also referred herein as to specific recombinant antigens from a pathogen, preferably a tick.

An "immunogenic fragment" of an antigen according to the present invention is a partial amino acid sequence of the antigen or a functional equivalent of such a fragment that also acts as an antigen, that is detected and bound by antigen-specific antibody or B-cell receptor. An immunogenic fragment of an antigen is shorter than the complete antigen and is preferably between about 10, 50 or 100 and about 1000 amino acids long, more preferably between about 10, 50 or 30 and about 500 amino acids long, even more preferably between about 50 and about 250 amino acids long.

"Antibodies" as used herein are polyclonal and/or monoclonal antibodies or fragments thereof, including recombinant antibody fragments, as well as immunologic binding equivalents thereof, which are capable of specifically binding to the pathogen proteins and/or to fragments thereof. The term "antibody" is used to refer to either a homogeneous molecular entity or a mixture such as a serum product made up of a plurality of different molecular entities. Recombinant antibody fragments may, e.g., be derived from a monoclonal antibody or may be isolated from libraries constructed from an immunized non-human animal.

The term "treatment" as used herein refers to the medical care given to a patient for a disease or injury. This term includes curing the disease but also ameliorating, mitigating, or reducing the symptoms of said disease. Thus, the term "therapeutic treatment" or "treatment" as used herein refers to bringing a body from a pathological state or disease back to its normal, healthy state. The term "therapeutic treatment" or "treatment" as used herein, also refers to the mitigation, amelioration, or reduction of the harmful effect of radiation in a subject, or in some manner reducing the symptoms associated with such effects. The term "prophylactic treatment" as used herein refers to preventing a pathological state.

The terms " fusion protein" and "chimeric protein" are considered synonymous and thus are used interchangeably herein. They refer to artificial (i.e., not natural occurring) proteins created through the joining of two or more genes which are originally coded for separate or same proteins. They thus comprise two or more domains that are originally coded by different genes in nature. The term "domain" refers to distinct functional and/or structural units in a protein, and they are responsible for a particular function or interaction, contributing to the overall role of a protein. Preferably, in the context of the present invention, a domain is a region of a protein's polypeptide chain that comprises one tick's protein or a fragment thereof.

## DESCRIPTION OF THE EMBODIMENTS

In **a first aspect,** the present invention provides a vaccine composition, also called herein "the vaccine composition of the invention", comprising at least one of the following antigens or proteins:

- the tick's Bm86 protein, an immunogenic fragment, or a functional equivalent thereof,
- the tick's Subolesin protein, an immunogenic fragment, or a functional equivalent thereof, and/or
- the tick's P0 protein, an immunogenic fragment, or a functional equivalent thereof.

The vaccine composition of the invention is capable of generating an immune response against ticks when the vaccine composition is inoculated to a mammal.

By "tick's Bm86 protein or antigen" is referred herein to a protein located on the luminal surface of the tick's midgut that, when it is blocked it causes the lysis of the tick's gut wall, interfering with blood digestion and egg production. Preferably, the tick's Bm86 protein comprises, consists, or consists essentially of SEQ ID NO: 5, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 5. Preferably, the tick's Bm86 protein is encoded by a nucleotide sequence that comprises, consists, or consists essentially of SEQ ID NO: 6, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 6. In the context of the present invention, an "immunogenic fragment or a functional equivalent of the tick's Bm86 protein" is referred to a protein or polypeptide capable of eliciting an immune response against the tick's Bm86 protein. Said protein or polypeptide may be longer or shorter than the tick's Bm86 protein as defined herein, or it may contain part of full sequence of the tick's Bm86 protein as defined herein, provided that it is capable of eliciting an immune response against said Bm86 protein.

By "tick's Subolesin protein or antigen" is referred herein to a protein that is located in the salivary glands of the ticks, and is associated with functions such as transcription, immunogenicity, gene expression, development, and physiology. Preferably, the tick's Subolesin protein comprises, consists, or consists essentially of SEQ ID NO: 1, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 1. Preferably, the tick's Subolesin protein is encoded by a nucleotide sequence that comprises, consists, or consists essentially of SEQ ID NO: 7, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 7. In the context of the present invention, an "immunogenic fragment or a functional equivalent of the tick's Subolesin protein" is referred to a protein or polypeptide capable of eliciting an immune response against the tick's Subolesin protein. Said protein or polypeptide may be longer or shorter than the tick's Subolesin protein as defined herein, or it may contain part of full sequence of the tick's Subolesin protein, provided that it is capable of eliciting an immune response against tick's Subolesin protein.

By "tick's P0 protein or antigen" is referred herein to is a protein located in the cellular cytoplasm of tick's cells and is essential for the assembly of the 60s ribosomal subunit. Preferably, the tick's P0 protein comprises, consists, or consists essentially of SEQ ID NO: 2, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 2. Preferably, the tick's P0 protein is encoded by a nucleotide sequence that comprises, consists, or consists essentially of SEQ ID NO: 8, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 8. In the context of the present invention, an "immunogenic fragment or a functional equivalent of the tick's P0 protein" is referred to a protein or polypeptide capable of eliciting an immune response against the tick's P0 protein. Said protein or polypeptide may be longer or shorter than the tick's P0 protein as defined herein, or it may contain part of full sequence of the tick's P0 protein, provided that it is capable of eliciting an immune response against tick's P0 protein.

In an embodiment, the vaccine composition comprises the tick's Bm86 protein, an immunogenic fragment, or a functional equivalent thereof, and the tick's Subolesin protein, an immunogenic fragment, or a functional equivalent thereof. In an embodiment, the vaccine composition comprises the tick's Bm86 protein, an immunogenic fragment, or a functional equivalent thereof, and the tick's P0 protein, an immunogenic fragment, or a functional equivalent thereof. In an embodiment, the vaccine composition comprises the tick's Subolesin protein, an immunogenic fragment, or a functional equivalent thereof, and the tick's P0 protein, an immunogenic fragment, or a functional equivalent thereof.

The antigens may be present in the vaccine composition of the invention in the form of separate, i.e. independent, proteins or polypeptides. Alternatively, the antigens may be present in the vaccine composition of the invention as part of a single polypeptide. By "part of a single polypeptide" is referred herein that they are bound by peptide bonds, so that they are part of a fusion or a chimeric protein.

In a preferred embodiment, the vaccine composition comprises the tick's Subolesin protein, an immunogenic fragment, or a functional equivalent thereof, and the tick's P0 protein, an immunogenic fragment, or a functional equivalent thereof, wherein said Subolesin and P0 proteins, fragments or functional equivalents, are part of a single polypeptide, forming a fusion protein. Preferably, the vaccine composition comprises a chimeric protein comprising or consisting of a first and a second domain, wherein the first domain comprises or consists of the tick's Subolesin protein, an immunogenic fragment, or a functional equivalent thereof; and the second domain comprises or consists of the tick's P0 protein, an immunogenic fragment, or a functional equivalent thereof. It is noted that the first domain may be placed in the N-terminal or C-terminal of the chimeric protein, and the same applies for the second domain. However, it is preferred that the first domain is located in the N-terminal region (i.e., the 5' region, preferably 5' end region) of the chimeric protein, and that the second domain is located downstream the first domain. Preferably, the second domain is located downstream, i.e., towards the C-terminal region, of the chimeric protein.

Hence, in a preferred embodiment, the vaccine composition comprises a chimeric protein antigen comprising:

- a first domain comprising or consisting of SEQ ID NO: 1, or an immunogenic fragment or functional equivalent thereof having at least 80%, 82%, 84%, 85%, 86%, 87%, 88%, 89%, 90%, 91%, 92%, 93%, 94%, 95%, 96%, 97%, 98%, 99% or 100% identity with SEQ ID NO: 1, and
- a second domain comprising or consisting of SEQ ID NO: 2, or an immunogenic fragment or functional equivalent thereof having at least 80%, 82%, 84%, 85%, 86%, 87%, 88%, 89%, 90%, 91%, 92%, 93%, 94%, 95%, 96%, 97%, 98%, 99% or 100% identity with SEQ ID NO: 2.

In a more preferred embodiment, the vaccine composition comprises a chimeric protein antigen comprising or consisting of a first and a second domain, wherein the first domain comprises or consists of SEQ ID NO: 1, and the second domain comprises or consists of SEQ ID NO: 2.

In an embodiment, the first and the second domains of the chimeric protein antigen are part of a single polypeptide and are bound, preferably covalently, by means of a linker. A "Linker" as used herein is a short peptide sequence that is located between the two domains of the chimeric protein. Linker peptides are placed to provide the two domains with movement flexibility. In the context of the present invention, the linker has at least one amino acid residue, preferably at least two consecutive amino acid residues, optionally 3, 4, 5, 6, 7, 8, 9, 10, 11, 12, 13, 14, 15, 16, 17, 18, 19, 20 or more amino acid residues. The linker peptide includes flexible linkers, rigid linkers, and in vivo cleavable linkers. Preferably, the linker comprises one or more Glycine and/or Serine amino acids. Most preferably, the linker comprises, consists, or consists essentially of SEQ ID NO: 4, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 4. Consequently, it is a preferred embodiment of the invention that the vaccine composition comprises a chimeric protein comprising or consisting of a first and a second domains, wherein the first domain comprises or consists of SEQ ID NO: 1, an immunogenic fragment, or a functional equivalent thereof; wherein the second domain comprises or consists of SEQ ID NO: 2, an immunogenic fragment, or a functional equivalent thereof; and wherein the first and second domains are bound by a linker, preferably a flexible linker, most preferably a linker comprising or consisting of SEQ ID NO: 4.

In a more preferred embodiment, the vaccine composition comprises a chimeric protein antigen comprising or consisting of SEQ ID NO: 3, or an immunogenic fragment or functional equivalent thereof having at least 80%, 82%, 84%, 85%, 86%, 87%, 88%, 89%, 90%, 91%, 92%, 93%, 94%, 95%, 96%, 97%, 98%, 99% or 100% identity with SEQ ID NO: 3.

In an embodiment, the vaccine composition comprises the three proteins described herein, i.e., the tick's Bm86 protein, an immunogenic fragment, or a functional equivalent thereof; the tick's Subolesin protein, an immunogenic fragment, or a functional equivalent thereof; and the tick's P0 protein, an immunogenic fragment, or a functional equivalent thereof. In an embodiment, the vaccine composition of the invention comprises the tick's Bm86 protein, an immunogenic fragment, or a functional equivalent thereof; the tick's Subolesin protein, an immunogenic fragment, or a functional equivalent thereof; and the tick's P0 protein, an immunogenic fragment, or a functional equivalent thereof, wherein the Subolesin protein and the P0 protein are in the form of a chimeric protein, i.e., comprised in a single polypeptide, and wherein the Bm86 protein is provided as a separate protein. Hence, preferably, the vaccine composition of the invention comprises at least two proteins:

- a first protein comprising or consisting of the tick's Bm86 protein, an immunogenic fragment, or a functional equivalent thereof, and
- a second protein that is a chimeric protein comprising or consisting of a first and a second domain, wherein the first domain comprises or consists of the tick's Subolesin protein, an immunogenic fragment, or a functional equivalent thereof; the second domain comprises or consists of the tick's P0 protein, an immunogenic fragment, or a functional equivalent thereof; and wherein, preferably, the first and second domains are connected or bound by a linker, preferably a flexible polypeptide linker.

In an embodiment, the vaccine composition comprises at least two proteins:

- a first protein comprising or consisting of the tick's Bm86 protein, an immunogenic fragment, or a functional equivalent thereof, wherein the tick's Bm86 protein comprises, consists, or consists essentially of SEQ ID NO: 5, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 5, and
- a second protein that is a chimeric protein comprising or consisting of a first and a second domain, wherein:

    i) the first domain comprises or consists of the tick's Subolesin protein, an immunogenic fragment, or a functional equivalent thereof; wherein the tick's Subolesin protein comprises, consists, or consists essentially of SEQ ID NO: 1, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 1,
    ii) the second domain comprises or consists of the tick's P0 protein, an immunogenic fragment, or a functional equivalent thereof; wherein the tick's P0 protein comprises, consists, or consists essentially of SEQ ID NO: 2, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 2, and
    iii) optionally, wherein the first and second domains are connected or bound by a linker, preferably a flexible polypeptide linker, preferably a linker that comprises, consists, or consists essentially of SEQ ID NO: 4, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 4.

In an embodiment, the vaccine composition comprises at least two proteins:

- a first protein comprising or consisting of the tick's Bm86 protein, wherein the tick's Bm86 protein comprises or consists of SEQ ID NO: 5, and
- a second protein that is a chimeric protein comprising or consisting of a first and a second domain, wherein:

    i) the first domain comprises or consists of the tick's Subolesin protein, wherein the tick's Subolesin protein comprises or consists of SEQ ID NO: 1,
    ii) the second domain comprises or consists of the tick's P0 protein, wherein the tick's P0 protein comprises or consists of SEQ ID NO: 2, and
    iii) optionally, wherein the first and second domains are connected or bound by a linker, preferably a flexible polypeptide linker, preferably a linker that comprises or consists of SEQ ID NO: 4.

In an embodiment, the vaccine composition comprises two proteins:

- a first protein comprising or consisting of the tick's Bm86 protein, an immunogenic fragment, or a functional equivalent thereof, wherein the tick's Bm86 protein comprises or consists of SEQ ID NO: 5, or a sequence with at least 80%, 82%, 84%, 85%, 86%, 87%, 88%, 89%, 90%, 91%, 92%, 93%, 94%, 95%, 96%, 97%, 98%, 99% or 100% sequence identity to SEQ ID NO: 5, and
- a second protein that is a chimeric protein that comprises or consists of SEQ ID NO: 3, or an immunogenic fragment or functional equivalent thereof having at least 80%, 82%, 84%, 85%, 86%, 87%, 88%, 89%, 90%, 91%, 92%, 93%, 94%, 95%, 96%, 97%, 98%, 99% or 100% identity with SEQ ID NO: 3.

In an embodiment, the vaccine composition comprises two proteins:

- a first protein comprising the tick's Bm86 protein, wherein the tick's Bm86 protein comprises or consists of SEQ ID NO: 5, and
- a second protein that is a chimeric protein that comprises SEQ ID NO: 3.

In an embodiment, the vaccine composition comprises two proteins:

- a first protein consisting of the tick's Bm86 protein of SEQ ID NO: 5, and
- a second protein that is a chimeric protein that consist of SEQ ID NO: 3.

In an embodiment, the proteins comprised in the vaccine composition are isolated antigens, preferably recombinant antigens. Preferably, the vaccine composition of the invention is a protein subunit vaccine. Preferably, the vaccine composition does not comprise other tick's derived immunogens, such as attenuated ticks, or dead ticks.

Preferably, the tick's protein or proteins comprised in the vaccine composition of the invention are capable of generating an immune response against ticks, when inoculated to a mammal. An "immunological response" or "immune response" to an antigen or composition is the development in a subject of an innate, humoral and/or a cellular immune response to an antigen present in the composition of interest. Preferably, said immune response is an enhanced immune response. The expression "enhanced immune response" when used for an immune response against the ticks antigens, such as an antibody response (e.g., neutralizing antigen-specific antibody response), a cytokine response, a CD8 T cell response (e.g., immunodominant CD8 T cell response), or a CD4 T cell response, refers to an increase in the immune response in a subject administered with a vaccine comprising at least one tick antigens (treated mammal) relative to the corresponding immune response observed from a mammal administered with a vaccine that does not comprise any tick antigens (control mammal).

Preferably, the immune response against ticks is a protective immune response. The expression "protective immune response" means that the vaccinated subject is able to control an infection caused by the ticks against which the vaccination was done. Usually, the subject having developed a "protective immune response" develops only mild to moderate clinical symptoms or no symptoms at all due to the tick's infection, and/or the number of ticks present in said vaccinated subject is reduced in comparison to a control or unvaccinated one. Usually, a subject having a "protective immune response" or "protective immunity" against tick will not die as a result of the infection with said tick. The term "infection" is herein understood, but not limited to, as the presence of replication of the tick on the subject's body where the ticks feed on the host at different developmental stages (nymphal & adults).

The presence of an immune response, either enhanced or protective immune response, can be measured in numerous ways known by the skilled artisan. The Examples provided below show a way of measuring or detecting the presence of an immune response, by performing ELISA assays or by performing challenges with the ticks in vaccinated animals. However, it is preferable, in the context of the present invention, that the presence of the immune response is measured by analysing one or more of the following parameters:

a) a reduction in the tick's number in the inoculated mammal in comparison to a control mammal,
b) a reduction in the tick's reproduction capacity in the inoculated mammal in comparison to a control mammal,
c) a reduction in the Teleogine Decay Coefficient (CRT) in the inoculated mammal in comparison to a control mammal,
d) a reduction in the Ovilaying Coefficient (CRO) in the inoculated mammal in comparison to a control mammal, and/or
e) a reduction in the Hatching ratio (CRE) in the inoculated mammal in comparison to a control mammal.

A "control mammal" is a mammal of the same species as the treated mammal, but that has not been treated with the vaccine composition of the invention. In an embodiment, the increase/decrease of those parameters are preferably a statistically significant increase/decrease. By "statistically significant increase" or "statistically significant decrease" is referred herein as the determination by an analyst that the increase or decrease, respectively, in any of the above parameters is not explainable by chance alone, and thus it is influenced or caused by the treatment with the composition of the invention. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the increase or decrease of any of the parameters defined above is statistically significant when a statistical test is performed to compare it to the basal level in a reference or untreated cell or subject, and wherein the resulting p-value of said statistical test is of 0.1 or lower, preferably 0.05, 0.01, 0.001 or lower.

Preferably, the presence of the immune response is measured by analysing the efficacy of the vaccine composition. In this context, the efficacy is measured as follows:

$$\% \text{ of efficacy} = 100 \times [(1-CRT*CRO*CRE)].$$

In an embodiment, the vaccine composition has an efficacy of at least 50%, 60%, 70%, 75%, 80%, 82%, 84%, 85%, 86%, 87%, 88%, 89%, 90%, 91%, 92%, 93%, 94%, 95%, 96%, 97%, 98%, 99% or 100% .

In a preferred embodiment, the subject animal that is treated with the vaccine composition is a mammal. Preferably, the mammal is an ungulate or a domestic animal. "Ungulate" refers to hooved animals such as cows, horses, sheep, and goats. "Domestic animal" includes, without limiting, canids, felids, and bovids, such as dogs, cats, guinea pig, rabbits, horses, goat, wild board, cattle, ferrets, porcine species such as pigs, piglets sows or gilts, and sheep. Preferably, the mammal is a bovine. "Bovine" refers to cattle and sheep. Most preferably, the mammal is selected from the list consisting of cows, bulls, oxen, and calves.

Preferably, the tick against which the immune response is generated by administering the vaccine composition of the invention is from the family *Ixodidae,* preferably from the genus *Rhipicephalus.* Most preferably, the tick is a Uruguayan variant belonging to the genus *Rhipicephalus microplus.*

The vaccine composition of the invention may further comprise a pharmaceutically acceptable carrier. The carrier suitable for preparing the vaccine in liquid form may include water, or an isotonic saline solution, that is to say, with a salt concentration equal to that of the physiological cellular medium, or an oil, or the culture liquid wherein the bacteria are cultured, or the mixtures thereof. The proteins comprised in the vaccine composition, which are the main active ingredient, can be incorporated into liposomes or other vehicles, such as nanoparticles, for use in a vaccine formulation, or may be conjugated to polysaccharides or other polymers.

Additionally, if it is desired, the vaccine composition can include other auxiliary substances or pharmaceutically acceptable excipients such as for example wetting agents, dispersant agents, emulsifying agents, buffer agents (for example phosphate buffer), stabilizing agents such as carbohydrates (for example glucose, sucrose, mannitol, sorbitol, starch or dextrans), or proteins (for example albumin, casein, bovine serum or skimmed milk).

Also, the vaccine composition may include a pharmaceutically acceptable adjuvant. "Adjuvant" is a substance used to enhance the immune response. Pharmaceutically acceptable adjuvants include but are not limited to: aluminum hydroxide, aluminum phosphate, aluminum oxide, muramyl dipeptides, vitamin E, squalene, squalene, saponins for example Quil A, QS-21, ginseng, zymosan, glucans, non-ionic block polymers, monophosphoryl lipid A, vegetable oils, complete or incomplete Freund's adjuvant, incomplete Freund's adjuvant, W/O, O/W, W/O/W type emulsions, Ribi adjuvant system (Ribi Inc.), heat-labile enterotoxin from E.coli (recombinant or otherwise), cholera toxin, dimethylaminoehtyldextran, dextrans or analogs or mixtures thereof. Preferably, the adjuvant is incomplete Freund's adjuvant.

The vaccines of the invention are typically prepared as parenteral vaccines in the form of solutions, emulsions or liquid suspensions. They can also be prepared in a solid form suitable to be dissolved or suspended in a liquid vehicle before injection.

Particularly, the vaccine is prepared in liquid form or in dry powder form, lyophilized, freeze dried, spray dried, or foam dried. The liquid vehicles which can be used for preparing the vaccine of the invention include, for example, water (in particular, water for injection), saline solution with a physiological salt concentration, or the culture liquid in which the bacteria are cultured.

In an alternative first aspect, the present invention provides a vaccine composition comprising an antibody or an antiserum that is reactive with the proteins defined above. Said antibody or antiserum can be obtained by vaccinating an animal with the vaccine composition of the first aspect or any of its embodiments. Said antibody or antiserum can also be used as a vaccine or as a medicament.

In a second aspect, the present invention refers to the use of the vaccine of the first aspect (including the alternative first aspect), or any of its embodiments, in therapy, or as a medicament. In an embodiment, said use comprises generating an immune response against ticks in a mammal, preferably a bovine. Also, the use in medicine can also involve the treatment and prevention of tick infections, or diseases or symptoms associated or caused by a t tick infection. For example, the use may be to reduce the number of ticks that are infecting the mammal, or to reduce the risk that said mammal becomes infected by ticks. Preferably, the vaccine of the invention is for use in a method of prevention and/or treatment of diseases in a mammal, preferably ungulates, preferably bovines.

Preferably, the use is in the treatment, prevention, amelioration or reduction of tick's infection or diseases that occur when the mammal is infected with the ticks. With "prevention" is meant keeping the disease from happening. With "amelioration" is meant an improvement in a patient's disease, or the activity of making an effort to correct, or at least make more acceptable said disease. With "reduction" or "mitigation" is meant decreasing the severity, seriousness or painfulness of the disease. The second aspect also involves methods for treatment or prevention of ticks infection in a mammal, preferably ungulate, most preferably a bovine.

In some embodiments, the vaccine composition defined herein is administered to a mammal, preferably ungulate, most preferably a bovine, susceptible to or at risk of infection to induce an immune response against the antigens present in said vaccine composition and thus enhance the animal's own immune response capabilities. Such an amount is defined to be an "immunogenically effective amount". Thus, in an embodiment, the vaccine composition of the first aspect (including the alternative first aspect), or any of its embodiments, is administered to a mammal, preferably ungulate, most preferably a bovine, in an immunogenically effective amount to prevent or treat a tick's infection. Preferably, the immunogenetically effective amount is about 50-200μg, preferably 100 μg, per antigen per dose.

In an embodiment, the vaccine composition is used to generate an immune response, preferably an enhanced and/or protective response against ticks. Preferably, said immune response is capable of causing one or more of:

a) a reduction in the tick's number in the inoculated mammal in comparison to a control mammal,
b) a reduction in the tick's reproduction capacity in the inoculated mammal in comparison to a control mammal,
c) a reduction in the Teleogine Decay Coefficient (CRT) in the inoculated mammal in comparison to a control mammal,
d) a reduction in the Ovilaying Coefficient (CRO) in the inoculated mammal in comparison to a control mammal, and/or
e) a reduction in the Hatching ratio (CRE) in the inoculated mammal in comparison to a control mammal,

when administered to the mammal. Preferably, the vaccine composition, when used according to the second aspect, achieves efficacy against a tick's infection, as explained above.

The vaccine composition of the invention can be administered by different routes of administration. Particular routes include but are not limited to oral, transdermal, transmucosal, intradermal, subcutaneous, intramuscular, intraperitoneal or intravenous route. Particularly they are administered by subcutaneous route. According to the desired duration and effectiveness of the treatment, the compositions according to the invention may be administered once or several times, also intermittently, for example on a daily basis for several days, weeks or months and in different dosages. Particularly, the vaccine compositions of the invention are administered several times. More particularly the vaccination plan comprises two doses, the second dose administered 3 weeks after the first one.

A variety of vaccination regimens may be effective in immunizing the mammal, preferably cattle and other bovines. For example, ungulate young and adults can both be vaccinated, preferably cows and calves. The first immunization with an antigen is usually called a prime immunization. Preferably, the immune response, with a single dose is capable of, or is characterized by being, sufficient to prevent a vaccinated subject from contracting an infection that leads to the morbidity and/or mortality of said subject, when infected by ticks.

As explained, the vaccine composition of the first aspect (including the alternative first aspect) can be used according to the second aspect as a prime immunization. However, a second immunization, also called boost immunization, can be given after the first immunization, to reinforced said first immunization. Boosting immunizations are generally administered once or multiple times weeks or months after administration of the priming composition, for example, about 1 or 2 weeks or 3 weeks, or 4 weeks, or 6 weeks, or 8 weeks, or 16 weeks, or 20 weeks, or 24 weeks, or 28 weeks, or 32 weeks or one to two years. Preferably, the initial boosting inoculation is administered 1-12 weeks or 2-12 weeks after priming, more preferably 1, 2, 4 or 8 weeks after priming. In a preferred embodiment, the initial boosting inoculation is administered 4 or 8 weeks after priming. In additional preferred embodiments, the initial boosting is conducted at least 2 weeks or at least 4 weeks after priming. In still another preferred embodiment, the initial boosting is conducted 4-12 weeks or 4-8 weeks after priming. Thus, the vaccine composition of the invention can be used as a prime, as a boost, or both (prime and boost). Preferably, the vaccine composition is administrated at least twice.

The proteins present in the vaccine compositions may be administrated together or separately in one or more immunizations. Preferably, the proteins or antigens are administered together, or at least simultaneously.

The second aspect also provides methods of prevention and/or treatment of diseases in a mammal, preferably a bovine, preferably cattle, most preferably selected from the list consisting of cows, bulls, oxen, or calves. More in particular for preventing and/or treating tick's infections or diseases caused by ticks, preferably by Rhipicephalus microplus. Thus, the vaccine of the first aspect can be administered in a mammal in need thereof in an immunologically effective amount in a method for preventing and/or treating tick's infection.

In **a third aspect,** the present invention also provides methods for detecting the presence or absence of ticks in a biological sample. This can be done by using the antibodies or antiserum against tick's proteins as defined in the alternative first aspect of the invention, since it will bind and thus detect ticks and/or tick's proteins present in a biological sample.

Also in a third aspect, the present invention provides methods for purifying and detecting antibodies against the proteins comprised in the vaccine composition of the invention. This can be done by using the proteins comprised in the vaccine composition, as defined under the first aspect or any of its embodiments, to bind, and thus detect, antibodies against ticks. Examples of useful assays is described below:
For instance, the pure proteins and antibodies disclosed here can be useful in immunoassays, such as ELISA, immunoblot analysis and agglutination assays. Western blot (immunoblot) analysis can also be used to detect the presence of antibodies to ticks in the sample. This technique is a reliable method for confirming the presence of antibodies against a particular protein in the sample. Additionally, both antibodies and antigens could be used for affinity chromatography.

The third aspect also comprises method for purifying or isolating the antigens comprised in the vaccine composition of the invention.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

In **a fourth aspect,** the invention also provides a vaccination kit characterized in that it comprises a container comprising the vaccine composition or the vaccine of the invention. Said vaccination kit may be further characterized in that it comprises an informative manual or leaflet which contains the information on administering said vaccine composition.

**SEQUENCE LISTING**

SEQ ID NO: 1: **Subolesin (amino acid sequence)**

MACATLKRTHDWDPLHSPSGRSPKRRRCMPLSPPPTRAHQIDPSPFGDVPPKLTSE
EIAANIREEMRRLQRRKQLCFQGADPESQHTSGLSSPVHRDQPLFTFRQVGLICERM
MKERESKIREEYDHVLSTKLAEQYDTFVKFTYDQIQKRFEGATPSYLS

**SEQ ID NO: 2: p0 (amino acid sequence)**
AAGGGAAAAKPEESKKEEAK

**SEQ ID NO: 3: Subolesin-p0, also called SPOS (amino acid sequence)**

MACATLKRTHDWDPLHSPSGRSPKRRRCMPLSPPPTRAHQIDPSPFGDVPPKLTSE
EIAANIREEMRRLQRRKQLCFQGADPESQHTSGLSSPVHRDQPLFTFRQVGLICERM
MKERESKIREEYDHVLSTKLAEQYDTFVKFTYDQIQKRFEGATPSYLSGGGGGSGGGG
SGGGGSAAGGGAAAAKPEESKKEEAK

**SEQ ID NO: 4: linker (amino acid sequence)**
GGGGSGGGGSGGGGS

**SEQ ID NO: 5: BM86s (amino acid sequence)**

ESNPSKGSCVCEASDDLTLQCKIKNDFATDCRNRGGTAKLRTDGFIGATCDCGEWG
AMNKTTRNCVPTTCLRPDLTCKDLCEKNLLQRDSRCCQGWNTANCSAAPPADSYCS
PGSPKGPDGQCKNACRTKEAGFVCKHGCRSTDKAYECTCPSGSTVAEDGITCKSISY
TVSCTVEQKQTCRPTEDCRVQKGTVLCECPWNQHLVGDTCISDCVDKKCHEEFMDC
GVYMNRQSCYCPWKSRKPGPNVNINECLLNEYYYTVSFT

**SEQ ID NO: 6: BM86s (nucleic acid sequence)**

TGAAAGTAACCCGAGCAAGGGTAGCTGCGTCTGCGAAGCATCGGACGATCTAAC
GCTACAATGCAAAATTAAAAATGACTTCGCAACTGACTGCCGAAACCGAGGTGGC
ACTGCTAAGTTGCGCACGGATGGGTTTATTGGCGCAACGTGTGACTGTGGTGAAT
GGGGTGCGATGAACAAGACCACACGGAACTGTGTCCCTACCACGTGTCTTCGTC
CCGACTTGACCTGCAAAGACCTCTGCGAGAAAAACCTGCTTCAAAGGGATTCTCG
TTGTTGTCAGGGGTGGAACACAGCAAACTGTTCAGCCGCTCCTCCAGCTGACTCC
TATTGCTCTCCTGGGAGCCCCAAAGGACCGGACGGACAGTGTAAAAATGCTTGCA
GGACGAAAGAAGCTGGGTTTGTCTGCAAGCATGGATGCAGGTCCACCGACAAGG
CGTACGAGTGCACGTGCCCGAGTGGCTCTACCGTCGCCGAAGATGGCATTACCT
GCAAAAGTATTTCGTACACAGTCAGCTGCACTGTTGAGCAAAAACAGACCTGCCG
CCCAACCGAAGACTGTCGTGTGCAGAAAGGAACTGTGTTGTGTGAGTGCCCGTG
GAATCAACATCTAGTGGGGGACACGTGCATAAGTGATTGCGTCGACAAGAAATGT
CACGAAGAATTTATGGACTGTGGCGTATATATGAATCGACAAAGCTGCTATTGTCC
ATGGAAATCAAGGAAGCCGGGCCCAAATGTCAACATCAATGAATGCCTACTGAAT
GAGTATTACTACACGGTGTCATTCACCC

**SEQ ID NO: 7: Subolesin (nucleic acid sequence)**

ATGGCTTGCGCAACATTAAAGCGGACACATGACTGGGACCCCTTGCACAGTCCGA
GTGGCAGATCGCCGAAGCGACGCCGATGTATGCCTCTGTCCCCGCCTCCTACAA
GGGCGCACCAGATCGATCCCTCGCCCTTCGGAGACGTGCCACCAAAGTTGACTT
CAGAGGAGATAGCAGCCAACATCCGTGAGGAGATGCGACGGCTACAACGGCGCA
AGCAGCTCTGTTTCCAGGGCGCTGACCCTGAATCCCAGCATACCAGTGGTCTCTC
GTCACCAGTGCATCGAGACCAGCCCCTGTTCACGTTCCGCCAGGTTGGGCTCATT
TGCGAACGAATGATGAAGGAGCGAGAGAGCAAGATACGGGAGGAGTATGACCAT
GTGCTATCTACCAAACTCGCAGAACAGTACGACACATTTGTTAAATTTACCTACGA
CCAAATTCAGAAGCGGTTTGAAGGTGCCACGCCCAGCTATTTGTCGTAA

**SEQ ID NO: 8: SPOS (nucleic acid sequence)**

```
ATGGCTTGCGCAACATTAAAGCGGACACATGACTGGGACCCCTTGCACAGTCCGA
GTGGCAGATCGCCGAAGCGACGCCGATGTATGCCTCTGTCCCCGCCTCCTACAA
GGGCGCACCAGATCGATCCCTCGCCCTTCGGAGACGTGCCACCAAAGTTGACTT
CAGAGGAGATAGCAGCCAACATCCGTGAGGAGATGCGACGGCTACAACGGCGCA
AGCAGCTCTGTTTCCAGGGCGCTGACCCTGAATCCCAGCATACCAGTGGTCTCTC
GTCACCAGTGCATCGAGACCAGCCCCTGTTCACGTTCCGCCAGGTTGGGCTCATT
TGCGAACGAATGATGAAGGAGCGAGAGAGCAAGATACGGGAGGAGTATGACCAT
GTGCTATCTACCAAACTCGCAGAACAGTACGACACATTTGTTAAATTTACCTACGA
CCAAATTCAGAAGCGGTTTGAAGGTGCCACGCCCAGCTATTTGTCGggcggcggcggc
agcggcggcggcggcagcggcggcggcggcagcgcggcgggcggcggcgcggcggcggcgaaaccggaaga
aagcaaaaaagaagaagcgaaataa
```

The invention is described below by the following examples, which must be considered as merely illustrative and in no case limiting of the scope of the present invention.

**EXAMPLES**

**Materials and Methods**

A sampling of native ticks from Uruguay was carried out, including specimens from the following departments: Tacuarembó, Treinta y Tres, Flores, Lavalleja, and Cerro Largo. In all studies conducted, the Mozo strain in the telogen stage was incorporated as the reference strain. This reference was provided by the Department of Parasitology of the Veterinary Laboratories Division (DILAVE) and is the reference strain for Uruguay since 1973.

The collected ticks were washed with 70% ethanol and rinsed with 1X PBS before tissue dissection under a microscope. The obtained tissues were the salivary gland, intestine, and ovary. Those used for RNA extraction were quickly removed and stored at -80°C until further use. RNA extraction was performed using TRIzol reagent as well as the Quick-RNA fungal/bacterial Microprep kit. The integrity of the extracted RNA was evaluated by 1% agarose gel electrophoresis.

Reverse transcription PCR (RT-PCR) was performed from the total RNA extracted from the intestine (Bm86 expression) and salivary glands (Subolesin expression) using the RevertAid Reverse Transcriptase kit (200 U/μl). This kit contains a DNA polymerase that synthesizes complementary DNA (cDNA) from single-stranded RNA. This technology allowed the generation of mRNA from different antigens along with their respective cDNA. In all cases, the actin gene (constitutive gene) was used as a control.

Using the generated cDNA, a polymerase chain reaction (PCR) was performed with primers previously designed to amplify the corresponding antigens. The amplifications were carried out in the SimpliAmp Thermal Cycler. The cycling conditions consisted of 2 minutes at 94°C, followed by 35 cycles of: 30 seconds at 94°C, 30 seconds at the annealing temperature of 53°C, and 1 minute at 72°C. Finally, there was a 7-minute extension step at 72°C.

The PCR products were analyzed by agarose gel electrophoresis (1.5%) along with a molecular weight marker (Gene Ruler DNA Ladder Mix). The obtained amplicons were sent for sequencing to the company Macrogen (Seoul, Korea). Once the sequences of the fragments corresponding to Bm86 and Subolesin antigens were obtained, they were edited using SnapGene software. This procedure involved removing unreliable base sequence ends. Bioinformatics analysis allowed us to conclude that there is a degree of polymorphism present in BM86, which is consistent with the expected results. Furthermore, the absence of polymorphism in Subolesin was confirmed.

The gene sequences were used for the construction of three plasmids: a) One for the expression of what we call BM86s, with a sequence based on BM86 with corresponding mutations according to the polymorphism found in Uruguayan tick species, b) another for the recombinant expression of S0, c) a final one for a chimeric protein composed of a DNA fragment from the gene encoding Subolesin protein and the DNA encoding a 20-amino acid peptide from the P0 protein. The two antigens are held together by a flexible linker with a sequence of $(GGGGS)_3$. This protein is referred to as SPOS.

## Synthesis of recombinant plasmids

For the recombinant expression of BM86, Subolesin, and SPOS, the pet28a(+) expression plasmid was used. The generation of the three recombinant plasmids was performed by GenScript. The chosen expression vector is characterized by having a promoter region recognized by T7 RNA polymerase regulated by the T7 promoter, so that upon addition of IPTG, T7 RNA polymerase is expressed from the bacterial chromosome and induces protein expression from the vector. The vector includes a sequence encoding a six-histidine tag, which becomes fused to the N-terminal region of the recombinant protein after translation, facilitating its localization by Western Blot and purification by affinity chromatography using Ni resins. Additionally, it contains the gene that confers resistance to kanamycin.

## Expression at laboratory and pilot scale

The expression of recombinant antigens was performed in *E. coli* bacteria (BL21 DE3) at both laboratory scale (flasks) and pilot scale (bioreactor).

The production of antigens at the laboratory scale was carried out in 1-liter flasks with 250 ml of Luria-Bertani (LB) medium. The expression conditions were as follows: growth at 37 °C and 200 rpm agitation. Bacterial growth was monitored by spectrophotometric measurements at an optical density (OD) of 600nm, and the culture induction was performed by adding the inducing agent IPTG (1 mM final concentration) at an OD of 0.6-0.8. The induction time was 3 hours at 37 °C.

The fermentation scaling-up was performed in a 5 L Sartorius biostat bioreactor. An alternative culture medium called ZYM-5052 was used, which allowed growth and autoinduction. The expression conditions for the three antigens were as follows: agitation at 250 rpm, temperature at 37 °C, and pH at 6.8. Bacterial growth curves were constructed by measuring the optical density (OD) at 1-hour intervals to determine the optimal induction time. In all cases, the culture was harvested by centrifugation at 5000 rpm.

## Antigen purification

To obtain our target antigens in purified form, the generated bacterial pellet was weighed and lysed using the following lysis buffer: phosphate-buffered saline (PBS) containing 1 mg/ml lysozyme, and 1 mM PMSF (10 mL/gram of pellet).

The mixture was incubated for 30 minutes at 4°C, followed by sonication of the sample on ice for 10 minutes (210 um amplitude capacity, 20% vibration amplitude, 1 min on and 1 min off). The sample was then centrifuged at 14000 rpm for one hour at 4°C.

The pellet containing inclusion bodies was prepared to verify the correct expression of the antigens using 12% SDS-PAGE gels.

Purification was performed as follows. 1.4 g of inclusion bodies containing the antigen were weighed and resuspended in 10 mL of lysis buffer. The mixture was left on a shaker at 37°C for 1 hour with minimal agitation (50 rpm). Subsequently, the mixture was centrifuged at 15000 rpm for 30 minutes. The insoluble fraction was solubilized in lysis buffer and loaded in a column containing 1 g of iminodiacetic acid-activated agarose loaded with $NiSO_4$ 100 mM (IDA-Ni), and equilibrated with the same lysis buffer without imidazole.

The column loaded with 10 mL of sample was shaken on a rolling shaker for 1 hour at 25°C. The percolate was then collected, and 10 mL of wash buffer was applied (lysis buffer containing 10 mM imidazole), and subsequently collected. Elution buffer 1 (250 mM imidazole) was added, shaken for 30 minutes at 25°C and the sample was collected. Elution buffer 2 (500 mM imidazole) was added, shaken for 30 minutes at 25°C and the sample was collected.

The samples eluted with 250 mM and 500 mM imidazole were pooled and used for gel filtration through FPLC using a HiLoad Superdex 200pg column (120 mL). A flow rate of 1 mL/min and detection at 280 nm were used. The column was equilibrated with two volumes of PBS. 2 ml of the applied sample, previously filtered with 0.45 $\mu$m and then 0.22 $\mu$m filters, were injected. Elution was carried out in PBS, where the first 40 mL (dead volume) and then the subsequent 80 mL were collected in 1 mL samples.

## Evaluation of immune response

In the initial stage, mice were used as the model organism to test the immune response. The immunization protocol for the following objective involved the use of 6 mice for each type of antigen. Two doses of 100 $\mu$g of antigen, along with incomplete Freund's adjuvant (IFA), were injected with a 15-day interval.

The analysis of humoral response was performed using the western blot technique. For this analysis, the antigens were separated using 12% SDS-PAGE and transferred to a nitrocellulose membrane. The obtained membranes were cut into strips and blocked overnight with PBS-TWEEN and 5% skim milk. Subsequently, they were incubated for 1 hour at 25°C with a 1/500 dilution of the obtained mouse sera (antigens and control). They were then washed with 0.5% PBS-TWEEN and incubated with the alkaline phosphatase-conjugated anti-IgG secondary antibody. Visualization was achieved using NBT BCIP.

Subsequently, direct experimentation was conducted on cattle. The immunization protocol for the following objective involved the use of 15 animals (3 animals per formulation) to evaluate the immunogenicity of the antigens. A single dose and two boosters were administered within a 60-day period, with 2 ml of 100 $\mu$g antigen along with incomplete Freund's adjuvant (IFA). The formulations used were: 1) BM86s, 2) Subolesin, 3) SPOS, 4) BM86s and SPOS, and 5) Control (PBS with adjuvant).

The results of the generated humoral response were evaluated using the indirect ELISA technique. For this analysis, the plate was sensitized with 100 $\mu$l of the antigens to be immunized, and incubated at 4 °C for 16 hours. A blocking solution of 0.5% PBS-TWEEN with 5% milk was added to each well, and 300 $\mu$l was added per well for 1 hour at 37 °C.

Subsequently, the obtained cattle sera were incubated at 37 °C for 1 hour, in a dilution range of 1/500 to 1/32000. An alkaline phosphatase-conjugated anti-IgG secondary antibody was then added at a dilution of 1/4000 and incubated for 45 minutes at 37 °C.

The indirect ELISA was developed using 10 mg of p-nitrophenyl phosphate (PNPP) in 10 ml of 100 mM Tris buffer, 5 mM MgCl2, and 100 mM NaCl at pH 9.5.

Three washes with 0.5% PBS-TWEEN were performed between each step of the ELISA.

A second trial was conducted to determine the percentage of biological protection in vaccinated animals, correlating it with the antibody titer generated by the immune response. The antibody titer was determined using the ELISA technique mentioned earlier.

The immunization stage took place at a DILAVE facility located in the Lavalleja department, Route 8 km 90, Aguas Blancas area (Uruguay). The immunization protocol was the same as mentioned before, with a total of 12 cattle being inoculated, 6 with BM86s and SPOS (antigen cocktail), and 6 as control (PBS and adjuvant).

Subsequently, 10 cattle were selected for the barn trial. The efficacy evaluation stage was conducted in barns at the DILAVE "Miguel C. Rubino" facility, Route 8 km 17. The experimental cattle came from the DILAVE field, free from ectoparasites and infectious diseases, without prior treatment with ectoparasiticidal agents.

The animals were challenged twice a week for three weeks with 100 mg of *R. microplus* larvae. The methodology was based on the technical procedure PR-PAR-02 "Determination of efficacy and residual activity of acaricide products."

In the barn trial, the collected ticks were counted, weighed, and 20 were randomly selected from each animal daily. They were incubated in a stove at 27°C and 90% relative humidity to study their reproductive behavior, recording the weight of the incubated ticks, the eggs, and the percentage of hatching.

To determine the percentage of biological protection, three parameters were analyzed:

Coefficient of Reduction in Engorgement Weight (CRT) CRT = Average weight of engorged ticks in the vaccinated group / Average weight of engorged ticks in the control group Coefficient of Reduction in Oviposition (CRO) CRO = Average weight of eggs laid by engorged ticks in the vaccinated group / Average weight of eggs laid by engorged ticks in the control group Coefficient of Reduction in Hatching (CRE) CRE = Percentage of egg hatching in the vaccinated group / Percentage of egg hatching in the control group

The coefficients were calculated by comparing the animals in the treated and control groups. The approval criterion was based on the following indicators: The Overall Efficacy in the parasite cycle should be equal to or greater than 50%.

**Results and Discussion**

The information obtained from the antigenic sequences allowed us to conclude the following: the degree of polymorphism present in Bm86, which is consistent with the expected results, and the absence of polymorphism in Subolesin.

The generation of the recombinant antigen BM86s, whose sequence matches the indigenous populations in our country, increase the levels of immune protection, thus reducing the failures in protection percentages when using foreign vaccines.

## Expression of Recombinant Antigens

Bacterial fermentations were carried out at the laboratory scale (flasks) and pilot scale (bioreactor) for the expression of recombinant antigens. In all cases, overexpression of our target antigens was observed compared to other proteins.

The antigens used in the formulation were expressed in the insoluble fraction as inclusion bodies. Presenting antigens in this form allows for a higher degree of protection, avoiding costly and complex processes such as purification, solubilization, and refolding.

An innovative aspect is the generation of a chimeric protein as an immunizing antigen. The following genetic construction involves a DNA fragment from the coding gene of the subolesin protein and the coding DNA for a 20-amino acid peptide from the P0 protein. The two antigens are kept together by a flexible linker.

## Evaluation of immune response

Analysis of post-immunized mouse sera revealed bands corresponding to the expected molecular weights of the recombinant antigens. No recognition was detected in the control samples (PBS with incomplete Freund's adjuvant). Thus, we conclude that immunization with different formulations elicits a robust immune response, as assessed by western blot, against our target antigens (Figure 5). The results of the humoral response generated in cattle were evaluated using the ELISA technique Fig. 6 shows the results obtained for the ELISA tests for each antigen and for the mixture of BM86s and SPOS (antigen cocktail). Good immune response values were obtained in all analyzed formulations. These results allowed us to conclude that immunization with different formulations triggers an immune response as a protective system. It was demonstrated that the response for the antigen cocktail (BM86s and SPOS) was significantly higher than that of the isolated recombinant proteins under the conditions studied (Figure 7). The response increased with the two consecutive boosters as observed in Figure 8.

The efficacy obtained for the protection of the antigenic cocktail was evaluated in cows after the immunization protocol providing an average of 70% (Figure 9). These results correlate with the immune response measured by ELISA (Figure 10 A) in which cows with an increased antibody titer (Abs 450 nm) showed better protection results. Moreover, the average antibody titer as evaluated by pooling the sera of vaccinated cows was significantly higher than the ELISA response of control cows vaccinated with PBS plus adjuvant.

## Conclusions and Innovations

The combination of BM86, Subolesin, and SPOS in an antigenic cocktail presents a promising alternative for the generation of new and more effective formulations for the control of tick infestations in cattle.

Our innovations are as follows:

1) Use of a recombinant antigen cocktail for tick vaccine with sequences adapted to target species.

2) Formulating a recombinant vaccine composed of multiple antigens (multivalent vaccine) generates different populations of antibodies in the host organism as a result of immunization, which can be associated with better levels of protection.

3) Use of a chimera protein antigen with a completely new design. The sequence consists of selected sections from two known antigens fused by a flexible loop sequence designed by us.

## References

1. Jaime Betancur Hurtado, O. & Giraldo-Rios, C. Economic and Health Impact of the Ticks in Production Animals. in Ticks and Tick-Borne Pathogens (IntechOpen, 2019). doi:10.5772/intechopen.81167

2. Fuente, J. de la et al. A ten-year review of commercial vaccine performance for control of tick infestations on cattle. Anim. Heal. Res. Rev. 8, 23-28 (2007).

3. Trentelman, J. J. A. et al. A combination of antibodies against Bm86 and Subolesin inhibits engorgement of Rhipicephalus australis (formerly Rhipicephalus microplus) larvae in vitro. Parasit. Vectors 12, 362 (2019).

4. Pereira, D. F. S. et al. Rhipicephalus microplus: An overview of vaccine antigens against the cattle tick. Ticks Tick. Borne. Dis. 13, 101828 (2022).

5. Parthasarathi, B. C. et al. Analysis of Genetic Diversity in Indian Isolates of Rhipicephalus microplus Based on Bm86 Gene Sequence. Vaccines 9, 194 (2021).

6. Contreras, M., Kasaija, P. D., Kabi, F., Mugerwa, S. & De la Fuente, J. The Correlation between Subolesin-Reactive Epitopes and Vaccine Efficacy. Vaccines 10, 1327 (2022).

**Claims**

1. A vaccine composition comprising an isolated chimeric protein comprising:

   - a first domain comprising SEQ ID NO: 1, or a functional equivalent thereof having at least 90% identity with SEQ ID NO: 1,
   - a second domain comprising SEQ ID NO: 2, or a functional equivalent thereof having at least 90% identity with SEQ ID NO: 2.

2. The vaccine composition according to claim 1, wherein the first domain consists of SEQ ID NO: 1 and the second domain consists of SEQ ID NO: 2.

3. The vaccine composition according to any one of claims 1 or 2, wherein the first and the second domains are connected by a peptide linker, preferably a linker comprising or consisting of SEQ ID NO: 4.

4. The vaccine composition according to any one of claims 1 to 3, wherein the chimeric protein consists of SEQ ID NO 3.

5. The vaccine composition according to any one of claims 1 to 4, further comprising a protein comprising SEQ ID NO: 5, or a functional equivalent thereof having at least 90% identity with SEQ ID NO: 5.

6. The vaccine composition according to any one of claims 1 to 5, comprising the proteins consisting of SEQ ID NO: 3 and 5.

7. The vaccine composition according to any one of claims 1 to 6, wherein the immune response against ticks results in one or more of:

   a) a reduction in the tick's number in the inoculated mammal in comparison to a control mammal,
   b) a reduction in the tick's reproduction capacity in the inoculated mammal in comparison to a control mammal,
   c) a reduction in the Teleogine Decay Coefficient (CRT) in the inoculated mammal in comparison to a control mammal,
   d) a reduction in the Ovilaying Coefficient (CRO) in the inoculated mammal in comparison to a control mammal, and/or
   e) a reduction in the Hatching ratio (CRE) in the inoculated mammal in comparison to a control mammal.

8. The vaccine composition according to any one of claims 1 to 7, wherein the tick is *Rhipicephalus microplus*.

9. The vaccine composition according to any one of claims 1 to 8, wherein the mammal is a bovine, preferably cattle, most preferably selected from the list consisting of cows, bulls, oxen, or calves.

10. The vaccine composition according to any one of claims 1 to 9, **for use** in a method of generating an immune response against ticks in a mammal.

11. The vaccine composition for use according to claim 10, wherein the tick is *Rhipicephalus microplus*.

12. The vaccine composition for use according to any one of claims 10 or 11, wherein the mammal is a bovine, preferably cattle, most preferably selected from the list consisting of cows, bulls, oxen, or calves.

13. The vaccine composition for use according to any one of claims 10 to 12, wherein the vaccine is administrated as a prime and/or as a boost.

**14.** A vaccination kit comprising the vaccine composition according to any one of claims 1 to 9.

**15.** An antibody or an antiserum that is reactive with the immunogenic proteins of any one of claims 1 to 9.

**Fig. 1**

A) BM86

B) Subolesin-P0

**Fig. 2**

**Fig. 3**

**Fig 4.**

**Fig. 5**

**A) BM86**

**B) Subolesin-P0**

**Fig.6**

**Fig. 7**

**Fig. 8**

**Fig. 9**

**Fig. 10**

# EUROPEAN SEARCH REPORT

**Application Number**

EP 24 20 6224

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HASSAN IBRAHIM A ET AL: "Cross protection induced by combined Subolesin-based DNA and protein immunizations against adult Haemaphysalis longicornis", VACCINE, ELSEVIER, AMSTERDAM, NL, vol. 38, no. 4, 4 November 2019 (2019-11-04), pages 907-915, XP085984567, ISSN: 0264-410X, DOI: 10.1016/J.VACCINE.2019.10.076 [retrieved on 2019-11-04] * paragraph [03.3]; figure 1A * * paragraph [03.6] * * paragraph [03.7]; figure 3 * * table 1 * * paragraph [04.2]; figure 1F * ----- | 1,3,7, 10,13-15 | INV. A61K39/00 C07K14/435 A61P33/14 A61P33/00 |
| X | WO 2014/154847 A1 (INTERVET INT BV [NL]; INTERVET INC [US]) 2 October 2014 (2014-10-02) * paragraph [2.2.1.] * * paragraph [2.3.2.]; table 1 * * sequences 1, 2 * ----- | 2,4-6,8, 9,11,12 | |

**TECHNICAL FIELDS SEARCHED (IPC)**

C07K
A61K

The present search report has been drawn up for all claims

| Place of search | Date of completion of the search | Examiner |
|---|---|---|
| The Hague | 26 March 2025 | Steffensen, Marie |

## ANNEX TO THE EUROPEAN SEARCH REPORT
## ON EUROPEAN PATENT APPLICATION NO.

EP 24 20 6224

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2025

| Patent document cited in search report | Publication date | Patent family member(s) | | Publication date |
|---|---|---|---|---|
| WO 2014154847 A1 | 02-10-2014 | AR | 095939 A1 | 25-11-2015 |
| | | AU | 2014242942 A1 | 24-09-2015 |
| | | BR | 112015024882 A2 | 10-10-2017 |
| | | CA | 2907748 A1 | 02-10-2014 |
| | | CN | 105228647 A | 06-01-2016 |
| | | EP | 2978443 A1 | 03-02-2016 |
| | | ES | 2643031 T3 | 21-11-2017 |
| | | JP | 6586077 B2 | 02-10-2019 |
| | | JP | 2016516739 A | 09-06-2016 |
| | | MX | 357431 B | 09-07-2018 |
| | | RU | 2015146326 A | 10-05-2017 |
| | | US | 2016051649 A1 | 25-02-2016 |
| | | WO | 2014154847 A1 | 02-10-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Economic and Health Impact of the Ticks in Production Animals. **JAIME BETANCUR HURTADO, O.** ; **GIRALDO-RIOS, C.** Ticks and Tick-Borne Pathogens. IntechOpen, 2019
- **FUENTE, J. et al.** A ten-year review of commercial vaccine performance for control of tick infestations on cattle. *Anim. Heal. Res. Rev.*, 2007, vol. 8, 23-28
- **TRENTELMAN, J. J. A. et al.** A combination of antibodies against Bm86 and Subolesin inhibits engorgement of Rhipicephalus australis (formerly Rhipicephalus microplus) larvae in vitro. *Parasit. Vectors*, 2019, vol. 12, 362

- **PEREIRA, D. F. S. et al.** Rhipicephalus microplus: An overview of vaccine antigens against the cattle tick. *Ticks Tick. Borne. Dis.*, 2022, vol. 13, 101828
- **PARTHASARATHI, B. C. et al.** Analysis of Genetic Diversity in Indian Isolates of Rhipicephalus microplus Based on Bm86 Gene Sequence. *Vaccines*, 2021, vol. 9, 194
- **CONTRERAS, M.** ; **KASAIJA, P. D.** ; **KABI, F.** ; **MUGERWA, S.** ; **DE LA FUENTE, J.** The Correlation between Subolesin-Reactive Epitopes and Vaccine Efficacy. *Vaccines*, 2022, vol. 10, 1327